# FASCICULE DE BREVET EUROPEEN

(11) **EP 1 382 348 B1**
(45) Date de publication et mention de la délivrance du brevet: **26.10.2005**
(21) Numéro de dépôt: 03016996.5
(22) Date de dépôt: 16.07.1997
(51) Int. Cl.: A61K 39/295, A61K 39/155, A61P 31/14

(54) **Formule de vaccin polynucléotidique notamment contre la pathologie respiratoire des bovins**
Polynukleotid-Impfstoff, vorzugsweise zur Behandlung der Atemkrankheit bei Rindern
Polynucleotide vaccine formula, particularly for treating bovine respiratory disease

(30) Priorité: 19.07.1996 FR 9609403
(43) Date de publication de la demande: 21.01.2004
(62) Demande divisionnaire de: 97934578.2
(73) Titulaire: MERIAL, 69007 Lyon (FR)
(72) Inventeur: Audonnet, Jean-Christophe, 69006 Lyon (FR); Bouchardon, Annabelle, 69007 Lyon (FR); Baudu, Philippe, 69290 Craponne (FR); Riviere, Michel, 69130 Ecully (FR)
(74) Mandataire: Nargolwalla, Cyra

(56) Documents cités:
- EP-A- 0 661 059
- WO-A-93/14207
- WO-A-95/20660
- CROWE J E JR: "Current approaches to the development of vaccines against disease caused by respiratory syncytial virus ( RSV ) and parainfluenza virus (PIV): A meeting report of the WHO Programme for Vaccine Development (Nyon, Switzerland, March 27, 1994)." VACCINE 13 (4). 415-421, 1995, XP0002028713
- HARTIKKA J. ET AL.: "An Improved Plasmid DNA Expression Vector for Direct Injection into Skeletal Muscle" HUMAN GENE THERAPY, vol. 7, no. 10, 20 juin 1996 (1996-06-20), pages 1205-1217, XP002050079

## Description

La présente invention a trait à une formule de vaccin permettant la vaccination des bovins notamment contre la pathologie respiratoire. Elle a également trait à une méthode de vaccination correspondante.

Tous les bovins sont porteurs de virus et de bactéries potentiellement pathogènes à des degrés très variables.

Les virus peuvent se multiplier quand l'immunité spécifique est affaiblie et quand il y a des lésions des voies respiratoires. Ils sont ensuite excrétés par l'animal et peuvent alors contaminer d'autres animaux.

Parmi les virus que l'on rencontre, on peut citer notamment le virus parainfluenza de type 3 (PI-3), de pathogénécité propre modérée, le virus respiratoire syncitial bovin (RSV) et l'herpès virus bovin (BHV) encore appelé virus de la rinotrachéite infectieuse bovine (IBR), de pathogénicités propres élevées.

Un autre virus particulièrement important pour son rôle immunodépresseur et ses effets néfastes sur la reproduction est le virus de la maladie des muqueuses ou pestivirus bovin (BVDV).

Ces virus se traduisent en général par une phase primaire d'hyperthermie, de syndrome grippal et de troubles respiratoires, avec des troubles digestifs (diarrhées) dans le cas de BVD. Cette phase peut s'accompagner d'une phase secondaire avec apparition de bronchopneumonies liées à des infections bactériennes, en particulier Pasteurella, pouvant entraîner la mort. Ce phénomène est exacerbé en particulier par l'immunodépression consécutive à l'infection par BVD ou par l'infection des macrophages par PI-3. D'autres symptômes encore peuvent apparaître, comme des avortements avec BVD et BHV.

Il paraît donc nécessaire de tenter de mettre au point une prévention efficace contre les principaux virus intervenant dans la pathologie respiratoire des bovins.

On a déjà proposé par le passé des associations de vaccins contre certains virus responsables de la pathologie respiratoire des bovins.

Les associations développées jusqu'à présent étaient réalisées à partir de vaccins inactivés ou de vaccins vivants et éventuellement de mélanges de tels vaccins. Leur mise en oeuvre pose des problèmes de compatibilité entre valences et de stabilité. Il faut en effet assurer à la fois la compatibilité entre les différentes valences de vaccin, que ce soit au plan des différents antigènes utilisés ou au plan des formulations elles-mêmes, notamment dans le cas où l'on combine à la fois des vaccins inactivés et des vaccins vivants. Il se pose également le problème de la conservation de tels vaccins combinés et aussi de leur innocuité notamment en présence d'adjuvant. Ces vaccins sont en général assez coûteux.

Les demandes de brevet WO-A-90 11092, WO-A-93 19183, WO-A-94 21797 et WO-A-95 20660 ont fait usage de la technique récemment développée des vaccins polynucléotidiques. On sait que ces vaccins utilisent un plasmide apte à exprimer dans les cellules de l'hôte l'antigène inséré dans le plasmide. Toutes les voies d'administration ont été proposées (intrapéritonéale, intraveineuse, intramusculaire, transcutanée, intradermique, mucosale, etc.). Différents moyens de vaccination peuvent également être utilisés, tels que ADN déposé à la surface de particules d'or et projeté de façon à pénétrer dans la peau de l'animal (Tang et al,. Nature 356, 152-154, 1992) et les injecteurs par jet liquide permettant de transfecter à la fois dans la peau, le muscle, les tissus graisseux et les tissus mammaires (Furth et al., Analytical Biochemistry, 205, 365-368, 1992).

Les vaccins polynucléotidiques peuvent utiliser aussi bien des ADN nus que des ADN formulés par exemple au sein de liposomes ou de lipides cationiques.

G.J.M: COX a déjà proposé la vaccination polynucléotidique contre l'herpès virus bovin de type 1 dans J. of Virology, Volume 67, n° 9, septembre 1993, 5664-5667. Les auteurs ont notamment décrit des plasmides intégrant les gènes gI (gB), gIII (gC) et gIV (gD).

Dans Vaccine, Volume 13, n° 4, 415-421, 1995, J.E. CROWE présente une revue générale sur les différentes méthodes de vaccination contre le virus respiratoire syncitial et contre le virus parainfluenza de type 3. Cette revue reprend l'ensemble des possibilités offertes par les techniques actuelles de vaccination et suggère simplement que la technologie de l'immunisation polynucléotidique pourrait être utile dans la stratégie d'immunisation contre RSV et PI-3. Aucune construction de plasmide ni résultat de vaccination des bovins contre ces virus n'est décrit dans ce document.

L'invention se propose donc de fournir une formule de vaccin multivalent permettant d'assurer une vaccination contre un certain nombre de virus pathogènes intervenant notamment dans la pathologie respiratoire des bovins et ainsi assurer une vaccination efficace contre cette pathologie.

Un autre objectif de l'invention est de fournir une telle formule de vaccin associant différentes valences tout en présentant tous les critères requis de compatibilité et de stabilité des valences entre elles.

Un autre objectif de l'invention est de fournir une telle formule de vaccin permettant d'associer différentes valences dans un même véhicule.

Un autre objectif de l'invention est de fournir un tel vaccin qui soit de mise en oeuvre aisée et peu coûteuse.

Un autre objectif encore de l'invention est de fournir une telle formule de vaccin et une méthode de vaccination des bovins qui permettent d'obtenir une protection multivalente avec un niveau élevé d'efficacité et de longue durée, ainsi qu'une bonne innocuité et une absence de résidus.

La présente invention a donc pour objet une formule de vaccin notamment contre la pathologie respiratoire des bovins, comprenant au moins trois valences de vaccin polynucléotidique comprenant chacune un plasmide intégrant, de manière à l'exprimer in vivo dans les cellules hôtes, un gène d'une valence de pathogène respiratoire bovin, ces valences étant choisies parmi le groupe consistant en virus herpès bovin, virus respiratoire syncitial bovin, virus de la maladie des muqueuses et virus parainfluenza de type 3, les plasmides comprenant, pour chaque valence, un ou plusieurs des gènes choisis parmi le groupe consistant en gB et gD pour le virus herpès bovin, F et G pour le virus respiratoire syncitial bovin, E2, C + E1 + E2 et E1 + E2 pour le virus de la maladie des muqueuses, HN et F pour le virus parainfluenza de type 3.

Par valence, dans la présente invention, on entend au moins un antigène assurant une protection contre le virus du pathogène considéré, la valence pouvant contenir, à titre de sous-valence, un ou plusieurs gènes naturels ou modifiés d'une ou plusieurs souches du pathogène considéré.

Par gène d'agent pathogène, on entend non seulement le gène complet, mais aussi les séquences nucléotidiques différentes, y compris fragments, conservant la capacité à induire une réponse protectrice. La notion de gène recouvre les séquences nucléotidiques équivalentes à celles décrites précisément dans les exemples, c'est-à-dire les séquences différentes mais codant pour la même protéine. Elle recouvre aussi les séquences nucléotidiques d'autres souches du pathogène considéré, assurant une protection croisée ou une protection spécifique de souche ou de groupe de souche. Elle recouvre encore les séquences nucléotidiques qui ont été modifiées pour faciliter l'expression in vivo par l'animal hôte mais codant pour la même protéine.

De préférence, la formule de vaccin selon l'invention comprend les quatre valences.

En ce qui concerne la valence BHV, on préfère mettre en oeuvre les deux gènes codant pour gB et gD, dans des plasmides différents ou dans un seul et même plasmide. Eventuellement, mais d'une façon moins préférée, on peut utiliser l'un ou l'autre de ces gènes.

Pour la valence RSV, on utilise de préférence les deux gènes G et F intégrés dans deux plasmides différents ou dans un seul et même plasmide. Eventuellement, mais de façon moins préférée, on peut utiliser le gène F seul.

Pour la valence BVD, on préférera utiliser un plasmide intégrant le gène E2. Eventuellement, mais de façon moins préférée, on peut utiliser un plasmide codant pour E1 et E2 ensemble ou pour l'ensemble constitué par C, E1 et E2.

Pour la valence PI-3, on préfère utiliser l'ensemble des deux gènes HN et F dans deux plasmides différents ou dans un seul et même plasmide. On peut aussi utiliser uniquement le gène HN.

Une formule de vaccin préférée selon l'invention comprend et assure l'expression des gènes gB et gD de BHV, G et F de RSV, E2 de BVD et HN et F de PI-3.

La formule de vaccin selon l'invention pourra se présenter sous un volume de dose compris entre 0,1 et 10 ml et en particulier entre 1 et 5 ml.

La dose sera généralement comprise entre 10 ng et 1 mg, de préférence entre 100 ng et 500 µg et plus préférentiellement encore entre 1 µg et 250 µg par type de plasmide.

On utilisera de préférence des plasmides nus, simplement placés dans le véhicule de vaccination qui sera en général de l'eau physiologique (NaCl 0,9 %), de l'eau ultrapure, du tampon TE, etc. On peut bien entendu utiliser toutes les formes de vaccin polynucléotidique décrites dans l'art antérieur.

Chaque plasmide comprend un promoteur apte à assurer l'expression du gène inséré sous sa dépendance dans les cellules hôtes. Il s'agira en général d'un promoteur eucaryote fort et en particulier d'un promoteur précoce du cytomégalovirus CMV-IE, d'origine humaine ou murine, ou encore éventuellement d'une autre origine telle que rat, cochon, cobaye.

De manière plus générale, le promoteur pourra être soit d'origine virale, soit d'origine cellulaire. Comme promoteur viral autre que CMV-IE, on peut citer le promoteur précoce ou tardif du virus SV40 ou le promoteur LTR du virus du Sarcome de Rous. Il peut aussi s'agir d'un promoteur du virus dont provient le gène, par exemple le promoteur propre au gène.

Comme promoteur cellulaire, on peut citer le promoteur d'un gène du cytosquelette, tel que par exemple le promoteur de la desmine (Bolmont et al., Journal of Submicroscopic Cytology and Pathology, 1990, 22, 117-122 ; et ZHENLIN et al., Gene, 1989, 78, 243-254), ou encore le promoteur de l'actine.

Lorsque plusieurs gènes sont présents dans le même plasmide, ceuxi-ci peuvent être présentés dans la même unité de transcription ou dans deux unités différentes.

La combinaison des différentes valences du vaccin selon l'invention peut être effectuée, de préférence, par mélange de plasmides polynucléotidiques exprimant le ou les antigènes de chaque valence, mais on peut également prévoir de faire exprimer des antigènes de plusieurs valences par un même plasmide.

L'invention a encore pour objet des formules de vaccin monovalent comprenant un ou plusieurs plasmides codant pour un ou plusieurs gènes de l'un des virus choisis parmi le groupe consistant en BRSV, BVD et PI-3, les gènes étant ceux décrits plus haut. En dehors de leur caractère monovalent, ces formules peuvent reprendre les caractéristiques énoncées plus haut en ce qui concerne le choix des gènes, leurs combinaisons, la composition des plasmides, les volumes de dose, les doses, etc.

Les formules de vaccin monovalent peuvent être utilisées (i) pour la préparation d'une formule de vaccin polyvalent tel que décrit plus haut, (ii) à titre individuel contre la pathologie propre, (iii) associées à un vaccin d'un autre type (entier vivant ou inactivé, recombinant, sous-unité) contre une autre pathologie, ou (iv) comme rappel d'un vaccin comme décrit ci-après.

La présente invention a en effet encore pour objet l'utilisation d'un ou de plusieurs plasmides selon l'invention pour la fabrication d'un vaccin destiné à vacciner les bovins primo-vaccinés au moyen d'un premier vaccin classique du type de ceux de l'art antérieur choisi notamment dans le groupe consistant en vaccin entier vivant, vaccin entier inactivé, vaccin de sous-unité, vaccin recombinant, ce premier vaccin présentant, c'est-à-dire contenant ou pouvant exprimer, le ou les antigènes codé(s) par le ou les plasmides ou antigène(s) assurant une protection croisée.

De manière remarquable, le vaccin polynucléotidique a un effet de rappel puissant se traduisant par une amplification de la réponse immunitaire et l'instauration d'une immunité de longue durée.

De manière générale, les vaccins de primo-vaccination pourront être choisis parmi les vaccins commerciaux disponibles auprès des différents producteurs de vaccins vétérinaires.

L'invention a aussi pour objet un kit de vaccination regroupant un vaccin de primo-vaccination tel que décrit ci-dessus et une formule de vaccin selon l'invention pour le rappel. Elle a aussi trait à une formule de vaccin selon l'invention accompagnée d'une notice indiquant l'usage de cette formule comme rappel d'une primo-vaccination telle que décrite ci-avant.

La présente invention a également pour objet une méthode de vaccination des bovins contre la pathologie respiratoire, comprenant l'administration de la formule de vaccin efficace telle que décrit plus haut. Cette méthode de vaccination comprend l'administration d'une ou de plusieurs doses de formule de vaccin, ces doses pouvant être administrées successivement dans un court laps de temps et/ou successivement à des moments éloignés l'un de l'autre.

Les formules de vaccin selon l'invention pourront être administrées, dans le cadre de cette méthode de vaccination, par les différentes voies d'administration proposées dans l'art antérieur pour la vaccination polynucléotidique et au moyen des techniques d'administration connues.

L'invention a encore pour objet la méthode de vaccination consistant à faire une primo-vaccination telle que décrite ci-dessus et un rappel avec une formule de vaccin selon l'invention.

Dans une forme de mise en oeuvre préférée du procédé selon l'invention, on administre dans un premier temps, à l'animal, une dose efficace du vaccin de type classique, notamment inactivé, vivant, atténué ou recombinant, ou encore un vaccin de sous-unité de façon à assurer une primo-vaccination, et, de préférence dans un délai de 2 à 6 semaines, on assure l'administration du vaccin polyvalent ou monovalent selon l'invention.

L'invention concerne aussi la méthode de préparation des formules de vaccin, à savoir la préparation des valences et leurs mélanges, telle qu'elle ressort de cette description.

L'invention va être maintenant décrite plus en détails à l'aide de modes de réalisation de l'invention pris en récérence aux dessins annexés.

### Liste des figures

- Figure N° 1 :: Plasmide pVR1012
- Figure N° 2 :: Séquence du gène BHV-1 ST gB
- Figure N° 3 :: Construction du plasmide pPB156
- Figure N° 4 :: Plasmide pAB087
- Figure N° 5 :: Plasmide pA8011
- Figure N° 6 :: Plasmide pAB012
- Figure N° 7 :: Plasmide pAB058
- Figure N° 8 :: Plasmide pAB059
- Figure N° 9 :: Plasmide pAB060
- Figure N° 10 :: Plasmide pAB071
- Figure N° 11 :: Plasmide pAB072

### Liste des séquences SEQ ID N°

- SEQ ID N° 1 :: Séquence du gène BHV-1 gB (souche ST)
- SEQ ID N° 2 :: Oligonucléotide PB234
- SEQ ID N° 3 :: Oligonucléotide PB235
- SEQ ID N° 4 :: Oligonucléotide AB162
- SEQ ID N° 5 :: Oligonucléotide AB163
- SEQ ID N° 6 :: Oligonucléotide A8026
- SEQ ID N° 7 :: Oligonucléotide AB027
- SEQ ID N° 8 :: Oligonucléotide AB028
- SEQ ID N° 9 :: Oligonucléotide AB029
- SEQ ID N°10 :: Oligonucléotide AB110
- SEQ ID N°11 :: Oligonucléatide AB111
- SEQ ID N°12 :: Ologonucléotide AB114
- SEQ ID N°13 :: Oligonucléotide AB115
- SEQ ID N° 14 :: Oligonucléotide AB116
- SEQ ID N°15 :: Oligonucléotide AB117
- SEQ ID N°16 :: Oligonucléotide AB130
- SEQ ID N°17 :: Oligonucléotide AB131
- SEQ ID N°18 :: Oligonucléotide AB132
- SEQ ID N°19 :: Oligonucléotide AB133

### EXEMPLES

### Exemple 1 : Culture des virus

Les virus sont cultivés sur le système cellulaire approprié jusqu'à obtention d'un effet cytopathique. Les systèmes cellulaires à utiliser pour chaque virus sont bien connus de l'homme du métier. Brièvement, des cellules sensibles au virus utilisé, cultivées en milieu minimum essentiel de Eagle (milieu "MEM) ou un autre milieu approprié, sont inoculées avec la souche virale étudiée en utilisant une multiplicité d'infection de 1. Les cellules infectées sont alors incubées à 37°C pendant le temps nécessaire à l'apparition d'un effet cytopathique complet (en moyenne 36 heures).

### Exemple 2 : Extraction des ADNs génomiques viraux

Après culture, le surnageant et les cellules lysées sont récoltées et la totalité de la suspension virale est centrifugée à 1000 g pendant 10 minutes à + 4°C pour éliminer les débris cellulaires. Les particules virales sont alors récoltées par ultracentrifugation à 400000 g pendant 1 heure à + 4°C. Le culot est repris dans un volume minimum de tampon (Tris 10 mM, EDTA 1 mM). Cette suspension virale concentrée est traitée par la protéinase K (100 µg/ml final) en présence de sodium dodecyl sulfate (SDS) (0,5% final) pendant 2 heures à 37°C. L'ADN viral est ensuite extrait avec un mélange de phénol/chloroforme, puis précipité avec 2 volumes d'éthanol absolu. Après une nuit à - 20°C, l'ADN est centrifugé à 10000 g pendant 15 minutes à + 4°C. Le culot d'ADN est séché, puis repris dans un volume minimum d'eau ultrapure stérile. Il peut alors être digéré par des enzymes de restriction.

### Exemple 3 : isolement des ARNs génomiques vitaux

Les virus à ARN ont été purifiés selon les techniques bien connues de l'homme du métier. L'ARN viral génomique de chaque virus a été ensuite isolé en utilisant la technique d'extraction "thiocyanate de guanidium/phénol-chloroforme" décrite par P. Chomczynski et N. Sacchi (Anal. Biochem. 1987. 162. 156-159).

### Exemple 4 : Techniques de biologie moléculaire

Toutes les constructions de plasmides ont été réalisées en utilisant les techniques standards de biologie moléculaire décrites par J. Sambrook *et al.* (*Molecular Cloning: A Laboratory Manual.* 2^{nd} Edition. Cold Spring Harbor Laboratory. Cold Spring Harbor. New York. 1989). Tous les fragments de restriction utilisés pour la présente invention ont été isolés en utilisant le kit "Geneclean" (BIO101 Inc. La Jolla, CA).

### Exemple 5 : Technique de RT-PCR

Des oligonucléotides spécifiques (comportant à leurs extrémités 5' des sites de restriction pour faciliter le clonage des fragments amplifiés) ont été synthétisés de telle façon qu'ils couvrent entièrement les régions codantes des gènes devant être amplifiés (voir exemples spécifiques). La réaction de transcription inverse (RT) et l'amplification en chaîne par polymérase (PCR) ont été effectuées selon les techniques standards (J. Sambrook *et al. Molecular Cloning: A Laboratory Manual.* 2^{nd} Edition. Cold Spring Harbor Laboratory. Cold Spring Harbor. New York. 1989). Chaque réaction de RT-PCR a été faite avec un couple d'amplimers spécifiques et en prenant comme matrice l'ARN génomique viral extrait. L'ADN complémentaire amplifié a été extrait au phénoi/chloroforma/alcool isoamylique (25:24:1) avant d'être digéré par les enzymes de restriction.

### Exemple 6 : plasmide pVR1012

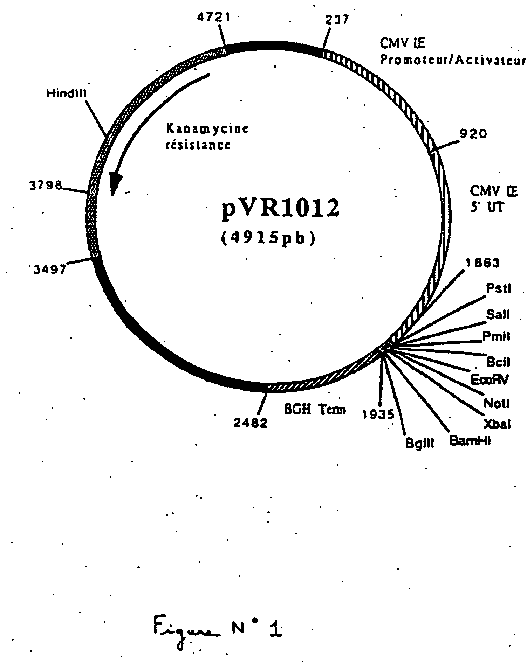

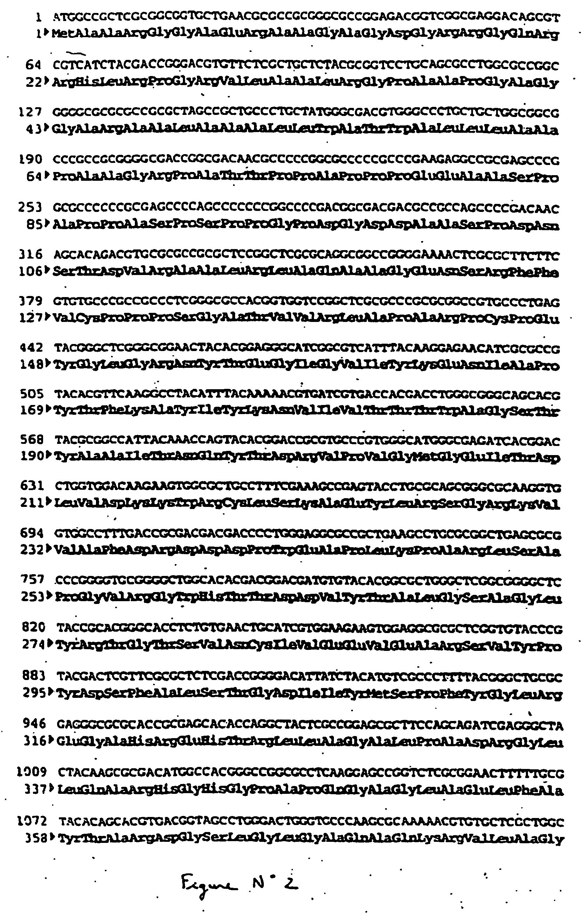

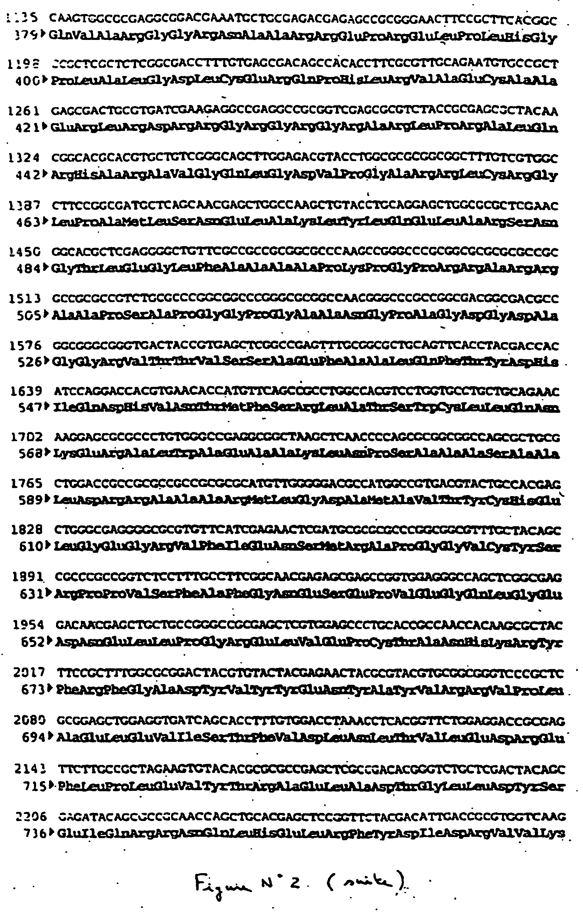

Le plasmide pVR1012 (Figure N° 1) a été obtenu auprès de Vical Inc. San Diego, CA, USA. Sa construction a été décrite dans J. Hartikka *et al.* (Human Gene Therapy. 1996. 7. 1205-1217).

### Exemple 7 : Construction du plasmide pPB156 (gène BHV-1 gB)

L'ADN génomique de l'herpèsvirus bovin BHV-1 (Souche ST) (Leung-Tack P. *et al.* Virology. 1994. 199. 409-421) a été préparé selon la technique décrite dans l'exemple 2 a été digéré par *Bam*HI. Après purification, le fragment BamHI-BamHl de 18 kpb a été cloné dans le vecteur pBR322 préalablement digéré par BamHI, pour donner le plasmide pIBR-4-BamHI (22 kpb).

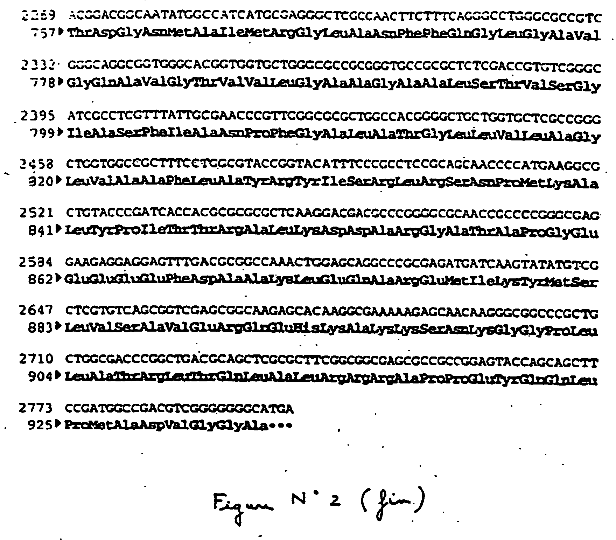

Le plasmide pIBR-4-BamHI a été ensuite digéré par *Sal*I pour libérer un fragment SalI-SalI de 6,6 kpb contenant le gène codant pour la glycoprotéine gB du BHV-1 (Figure N° 2 et SEQ ID N° 1). Ce fragment a été cloné dans le vecteur pBR322, préalablement digéré par *Sal*I, pour donner le plasmide pIBR-6,6-SalI (10,9 kpb).

Le plasmide pIBR-6,6-SalI a été digéré par *Nhe*I et *Bgl*II pour libérer un fragment

Nhel-Bglll de 2676 pb contenant le gène codant pour la glycoprotéine gB de l'herpèsvirus bovin (BHV-1) (fragment A).

Une réaction de PCR a été réalisée avec l'ADN génomique de l'herpésvirus bovin (BHV-1) (Souche ST) et avec les oligonucléotides suivants:
PB234 (30 mer) (SEQ ID N° 2) 5'TTGTCGACATGGCCGCTCGCGGCGGTGCTG 3'
PB235 (21 mer) (SEQ ID N° 3) 5'GCAGGGCAGCGGCTAGCGCGG 3' pour isoler la partie 5' du gène codant pour la glycoprotéine gB du BHV-1.

Après purification, le produit de PCR de 153 pb a été digéré par *Sal*I et *Nhe*I pour isoler un fragment SalI-NheI de 145 pb (fragment B).

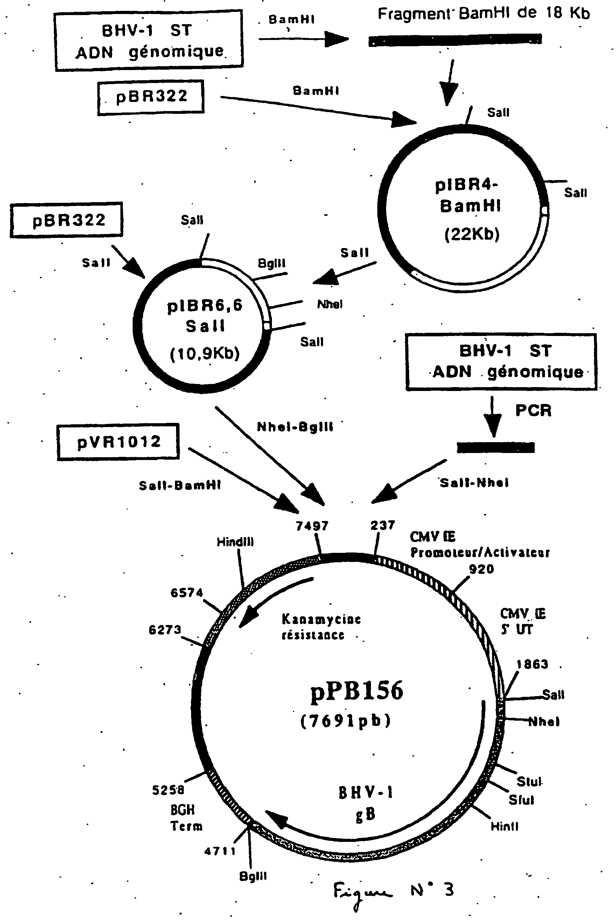

Les fragments A et B ont été ligaturés ensemble avec le vecteur pVR1012 (exemple 6), préalablement digéré avec *Sal*I et *BamH*I, pour donner le plasmide pPB158 (7691 pb) (Figure. N° 3).

### Exemple 8 : Construction du plasmide pAB087 (gène BHV-1 gD)

Une réaction de PCR a été réalisée avec l'ADN génomique de l'horpèsvirus bovin (BHV-1) (Souche ST) (P. Leung-Tack *et al*. Virology, 1994. 199. 409-421), préparé selon la technique décrite dans l'exemple 2 et avec les oligonucléotides suivants:
AB162 (31 mer) (SEQ ID N° 4) 5'AAACTGCAGATGCAAGGGCCGACATTGGCCG 3'
AB163 (27 mer) (SEQ ID N° 5) 5'ATCTTGTACCATATGACCGTGGCGTTG 3' pour amplifier la partie 5' du gène codant pour la glycoprotéine gD de l'herpèsvirus bovin (BHV-1) (N° d'accès séquence GenBank = L26360) sous la forme d'un fragment PCR de 338 pb. Après purification, ce fragment a été digéré par *Pst*I et *Nde*I pour isoler un fragment PstI-NdeI de 317 pb (fragment A).

Le plasmide pBHV001 (P. Leung-Tack *et al*. Virology. 1994. 199. 409-421.) a été digéré par *Nde*l et *Sty*I pour libérer un fragment de 942 pb contenant la partie 3' du gène codant pour la glycoprotéine gD du 8HV-1 (fragment B).

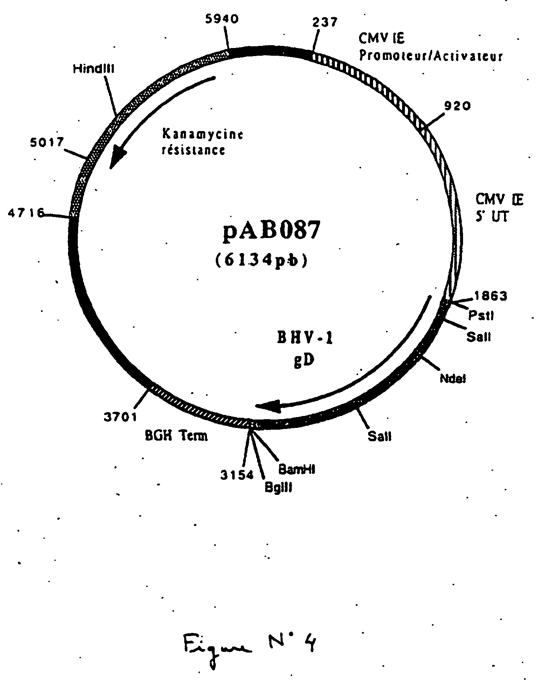

Les fragments A et B ont été ligaturés ensemble avec le vecteur pVR1012 (exemple 6), préalablement digéré avec *Pst*I et *Xbal,* pour donner le plasmide pAB087 (6134 pb) (Figure N° 4).

### Exemple 9 : Construction du plasmide pAB011 (gène BRSV F)

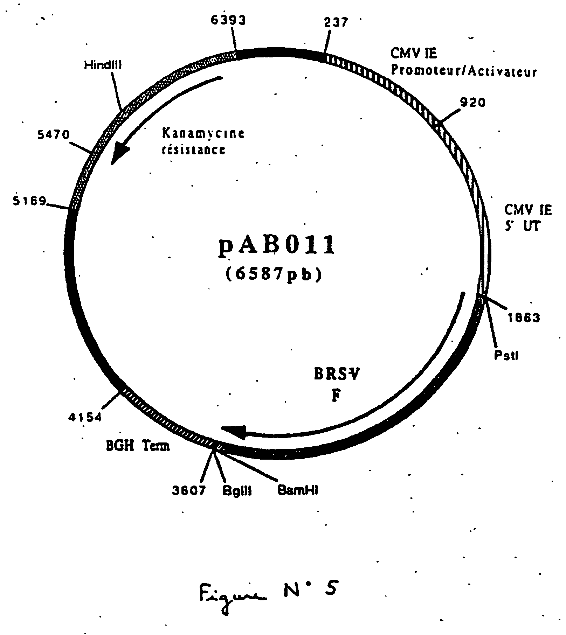

Une réaction de RT-PCR selon la technique décrite dans l'exemple 5 a été réalisée avec l'ARN génomique du virus respiratoire syncytial bovin (BRSV) (Souche 391-2) (R. Lerch *et al*. Virology. 1991. 181. 118-131), préparé comme indiqué dans l'exemple 3, et avec les oligonucléotides suivants:
AB026 (33 mer) (SEQ ID N° 6). 5'AAAACTGCAGGGATGGCGGCAACAGCCATGAGG 3'
AB027 (31 mer) (SEQ ID N° 7) 5'CGCGGATCCTCATTTACTAAAGGAAAGATTG 3' pour isoler le gène codant pour la glycoprotéine de fusion F (BRSV F) sous la forme d'un fragment PCR de 1734 pb. Après purification, ce fragment a été digéré par *Pst*I et *Bam*HI pour isoler un fragment PstI-BamHI de 1717 pb. Ce fragment a été ligaturé avec le vecteur pVR1012 (exemple 6), préalablement digéré avec *Pst*I et *Bsm*HI, pour donner le plasmide pAB011 (6587 pb) (Figure N° 5).

### Exemple 10 : Construction du plasmide pAB012 (gène BRSV G)

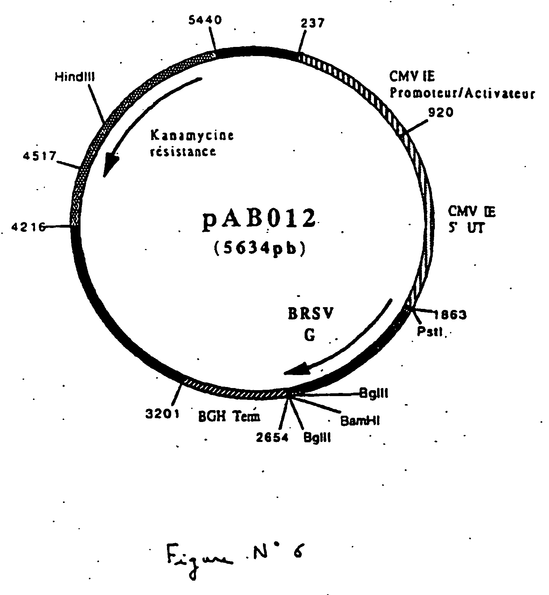

Une réaction de RT-PCR selon la technique décrite dans l'exemple 5 a été réalisée avec l'ARN génomique du virus respiratoire syncytial bovin (BRSV) (Souche 391-2) (R. Lerch *et al.* J. Virology. 1990. **64**. 5559-5569) et avec les oligonucléotides suivants:
AB028 (32 mer) (SEQ ID N° 8) 5'AAAACTGCAGATGTCCAACCATACCCATCATC 3'
AB029 (35 mer) (SEQ ID N° 9) 5'CGCGGATCCCTAGATCTGTGTAGTTGATTGATTTG 3' pour isoler le gène codant pour la protéine G (BRSV G) sous la forme d'un fragment PCR de 780 pb. Après purification, ce fragment a été digéré par *Pst*I et *Bam*HI pour isoler un fragment Pstl-BamHl de 763 pb. Ce fragment a été ligaturé avec le vecteur pVR1012 (exemple 6), préalablement digéré avec *Pst*I et *Bam*HI, pour donner le plasmides pAB012 (5634 pb) (Figure N° 6).

### Exemple 11 : Construction du plasmide pAB058 (gène BVDV C)

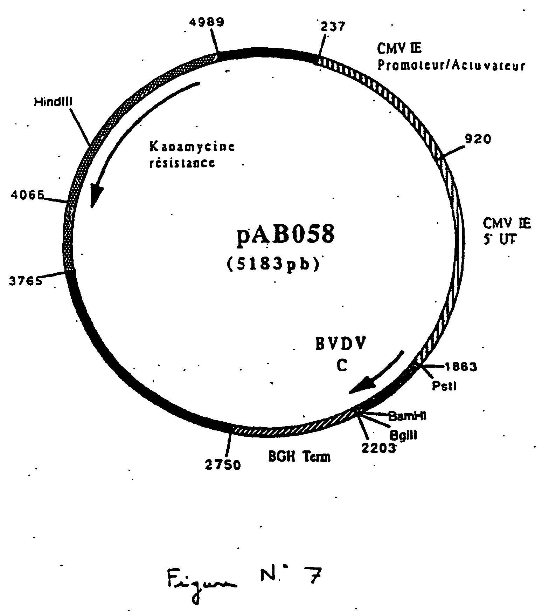

Une réaction RT-PCR selon la technique décrite dans l'exemple 5 a été réalisée avec l'ARN génomique du virus de la diarrhée virale bovine (BVDV) (Souche Osloss) (L. De Moerlooze *et al.* J. Gen. Virol. 1993. 74. 1433-1438), préparé selon la technique décrite dans l'exemple 3 et avec les oligonucléotides suivants:
AB110 (35 mer) (SEQ ID N° 10) 5'AAAACTGCAGATGTCCGACACAAAAGCAGAAGGGG 3'
AB111 (47 mer) (SEQ ID N° 11) 5'CGCGGATCCTCAATAAAAATCATTCCCACTGCGACTTGAAACAAAAC 3' pour amplifier un fragment de 342 pb contenant le gène codant pour la protéine de capside C du virus BVDV. Après purification, le produit de RT-PCR a été digéré par *Pst*I et *Bam*HI pour donner un fragment PstI-BamHI de 324 pb. Ce fragment a été ligaturé avec le vecteur pVR1012 (exemple 6), préalablement digéré avec *Pst*I et *Bam*HI, pour donner le plasmide pAB058 (5183 pb) (Figure N° 7).

### Exemple 12 : Construction du plasmide pAB059 ("gène" BVDV E1)

Une réaction RT-PCR selon la technique décrite dans l'exemple 5 a été réalisée avec l'ARN génomique du virus de la diarrhée virale bovine (BVDV) (Souche Osloss) (L. De Moerlooze *et al.* J. Gen. Virol. 1993. 74. 1433-1438) et avec les oligonucléotides suivants:
AB114 (32 mer) (SEQ ID N° 12) 5'ACGCGTCGACATGAAGAAACTAGAGAAAGCCC 3'
AB115 (33 mer) (SEQ ID N° 13) 5'CGCGGATCCTCAGCCGGGTTTGCAAACTGGGAG 3' pour isoler la séquence codant pour la protéine E1 du virus BVDV sous la forme d'un fragment PCR de 1381 pb. Après purification, ce fragment a été digéré par *Sal*I et *Bam*HI pour donner un fragment SalI-BamHI de 1367 pb.

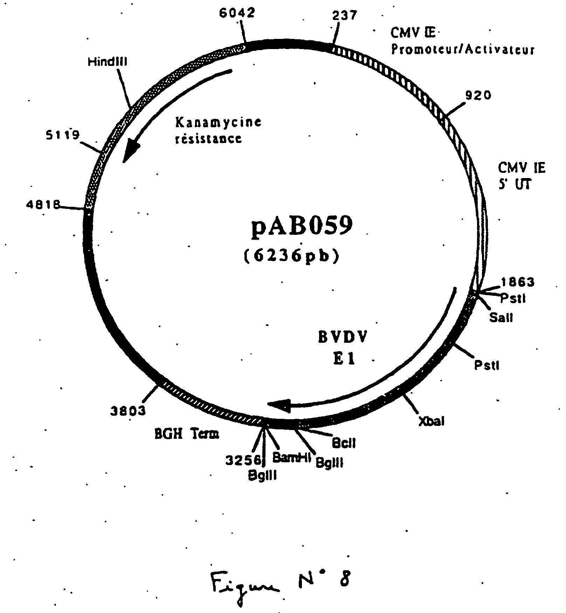

Ce fragment a été ligaturé avec le vecteur pVR1012 (exemple 6), préalablement digéré avec *Sal*I et *Bam*HI, pour donner le plasmide pAB059 (6236 pb) (Figure N° 8).

### Exemple 13 : Construction du plasmide pAB060 ("gène" BVDV E2)

Une réaction RT-PCR selon la technique décrite dans l'exemple 5 a été réalisée avec l'ARN génomique du virus de la diarrhée virale bovine (BVDV) (Souche Osloss) (L. De Moerlooze *et al.* J. Gen. Virol. 1993. 74. 1433-1438) et avec les oligonucléotides suivants:
AB116 (36 mer) (SEQ ID N° 14) 5'ACGCGTCGACATGACGACTACTGCATTCCTGGTATG 3'
AB117 (33 mer) (SEQ ID N° 15) 5'CGCGGATCCTCATTGACGTCCCGAGGTCATTTG 3' pour isoler la séquence codant pour la protéine E2 du virus BVDV sous la forme d'un fragment PCR de 1252 pb. Après purification, ce fragment a été digéré par *Sal*I et *Bam*HI pour donner un fragment SalI-BamHI de 1238 pb.

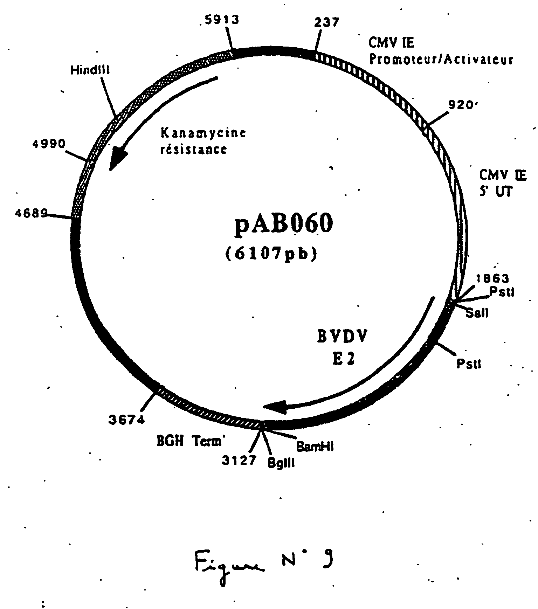

Ce fragment a été ligaturé avec le vecteur pVR1012 (exemple 6), préalablement digéré avec *Sal*I et *Bam*HI, pour donner le plasmide pAB060 (6107 pb) (Figure N° 9).

### Exemple 14 : Construction du plasmide pAB071 (gène BPIV HN)

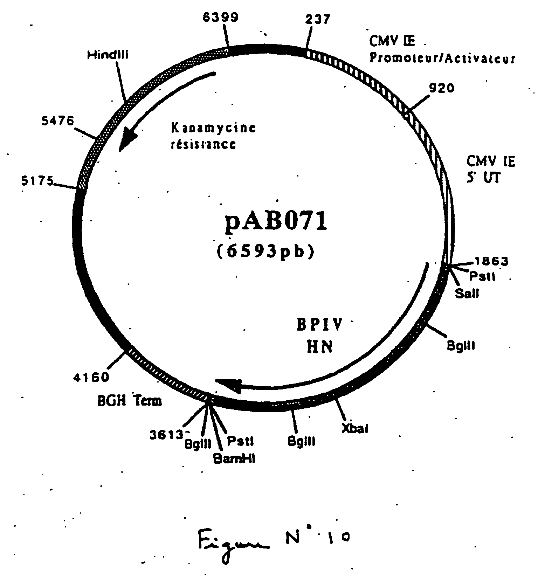

Une réaction de RT-PCR selon la technique décrite dans l'exemple 5 a été réalisée avec l'ARN génomique du virus parainfluenza bovin de type 3 (P13 = BPIV) et avec les oligonucléotides suivants:
AB130 (33 mer) (SEQ ID N° 16) 5' TTTGTCGACATGGAATATTGGAAACACACAAAC 3'
AB131 (33 mer) (SEQ ID N° 17) 5'TTTGGATCCTTAGCTGCAGTTTTTCGGAACTTC 3' pour isoler le gène codant pour la glycoprotéine HN du BPIV (séquence du gène HN déposée par H. Shibuta en 1987. N° d'accès de la séquence sur GenBank = Y00115) sous la forme d'un fragment PCR de 1737 pb. Après purification, ce fragment a été digéré par *Sal*I et *Bam*HI pour isoler un fragment SalI-BamHI de 1725 pb. Ce fragment a été ligaturé avec le vecteur pVR1012 (exemple 6), préalablement digéré avec *Sal*I et *Bam*HI, pour donner le plasmide pAB071 (6593 pb) (Figure N° 10).

### Exemple 15 : Construction du plasmide pAB072 (gène BPlV F)

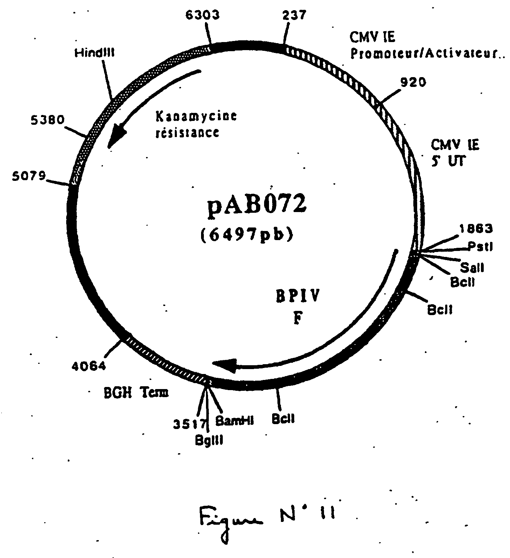

Une réaction de RT-PCR selon la technique décrite dans l'exemple 5 a été réalisée avec l'ARN génomique du virus parainfluenza bovin de type 3 (PI3 = BPIV) et avec les oligonucléotides suivants:
AB132 (30 mer) (SEQ ID N° 18) 5'TTTGTCGACATGATCATCACAAACACAATC 3'
AB133 (30 mer) (SEQ ID N° 19) 5'TTTGGATCCTCATTGTCTACTTGTTAGTAC 3' pour isoler le gène codant pour la protéine F du BPIV (séquence du gène F déposée par H. Shibuta en 1987. N° d'accès de la séquence sur GenBank = Y00115) sous la forme d'un fragment PCR de 1641 pb. Après purification, ce fragment a été digéré par *Sal*I et *Bam*HI pour isoler un fragment SalI-BamHI de 1629 pb. Ce fragment a été ligaturé avec le vecteur pVR1012 (exemple 6), préalablement digéré avec *Sal*I et *Bam*HI, pour donner le plasmide pAB072 (6497 pb) (Figure N° 11).

### Exemple 16 : Préparation et purification des plasmides

Pour la préparation des plasmides destinés à la vaccination des animaux, on peut utiliser toute technique permettant d'obtenir une suspension de plasmides purifiés majoritairement sous forme superenroulée. Ces techniques sont bien connues de l'homme de l'art. On peut citer en particulier la technique de lyse alcaline suivie de deux ultracentrifugations successives sur gradient dé chlorure de césium en présence de bromure d'éthidium telle que décrite dans J. Sambrook *et al.* (*Molecular Cloning: A Laboratory Manual.* 2^{nd} Edition. Cold Spring Harbor Laboratory. Cold Spring Harbor. New York. 1989). On peut se référer également aux demandes de brevet PCT WO 95/21250 et PCT WO 96/02658 qui décrivent des méthodes pour produire à l'échelle industrielle des plasmides utilisables pour la vaccination. Pour les besoins de la fabrication des vaccins (voir exemple 17), les plasmides purifiés sont resuspendus de manière à obtenir des solutions à haute concentration ( > 2 mg/ml) compatibles avec le stockage. Pour ce faire, les plasmides sont resuspendus soit en eau ultrapure, soit en tampon TE (Tris-HCl 10 mM; EDTA 1 mM, pH 8,0).

### Exemple 17 : Fabrication des vaccins associés

Les divers plasmides nécessaires à la fabrication d'un vaccin associé sont mélangés à partir de leurs solutions concentrées (exemple 16). Les mélanges sont réalisés de telle manière que la concentration finale de chaque plasmide corresponde à la dose efficace de chaque plasmide. Les solutions utilisables pour ajuster la concentration finale du vaccin peuvent être soit une solution NACI à 0,9 % , soit du tampon PBS.
Des formulations particulières telles que les liposomes, les lipides cationiques, peuvent aussi être mises en oeuvre pour la fabrication des vaccins.

### Exemple 18 : Vaccination des bovins

Les bovins sont vaccinés avec des doses de 100 µg, 250 µg ou 500 µg par plasmide. Les injections sont réalisées à l'aiguille par voie intramusculaire soit au niveau du muscle *gluteus*, soit au niveau des muscles du cou. Les doses vaccinales sont administrées sous des volumes compris entre 1 et 5 ml.

## Revendications

1. Vaccin bovin comprenant un plasmide contenant un gène du virus parainfluenza de type 3 (PI-3), ce gène étant choisi dans le groupe consistant en HN et F, et un véhicule approprié.

2. Vaccin selon la revendication 1, dans lequel le plasmide comprend les deux gènes HN et F.

3. Vaccin selon la revendication 1, qui comprend un plasmide contenant HN et un plasmide contenant F.

4. Vaccin selon l'une quelconque des revendications 1 à 3, dans lequel le plasmide comprend un promoteur choisi dans le groupe consistant en promoteur CMV-IE, promoteur précoce SV40, promoteur tardif SV40, promoteur LTR du virus du sarcome de Rous et promoteur d'un gène de cytosquelette.

5. Vaccin selon la revendication 4, dans lequel le promoteur est un promoteur CMV IE.

6. Vaccin selon la revendication 4, dans lequel le promoteur d'un gène du cystosquelette est le promoteur de la desmine ou le promoteur de l'actine.

7. Vaccin selon l'une quelconque des revendications 1 à 6, **caractérisé en ce qu'**il comprend en outre un plasmide contenant et exprimant un gène d'un autre pathogène respiratoire bovin.

8. Vaccin selon l'une quelconque des revendications 1 à 6, **caractérisé en ce qu'**il comprend en outre deux plasmides choisis dans le groupe consistant en un plasmide contenant le gène gB ou gD du virus herpès bovin, un plasmide contenant le gène E2, C + E1 + E2 et E1+E2 du virus de la maladie des muqueuses et un plasmide contenant le gène F ou G du virus respiratoire syncitial bovin.

9. Vaccin selon l'une quelconque des revendications 1 à 8, **caractérisé en ce qu'**il comprend de 10 ng à 1 mg, de préférence de 100 ng à 500 µg plus préférentiellement encore de 1 µg à 250 µg de chaque plasmide.

10. Utilisation d'un ou de plusieurs plasmides tels que décrits dans l'une quelconque des revendications 1 à 9, pour la fabrication d'un vaccin contre le virus parainfluenza de type 3 (PI-3) destiné à vacciner des bovins primo-vaccinés au moyen d'un premier vaccin choisi dans le groupe consistant en vaccin entier vivant, vaccin entier inactivé, vaccin de sous-unité, vaccin recombinant, ce premier vaccin présentant le ou les antigène(s) codé(s) par le ou les plasmide(s) ou antigène(s) assurant une protection croisée.

11. Kit de vaccination regroupant un vaccin polynucléotidique selon l'une quelconque des revendications 1 à 9 et un premier vaccin choisi dans le groupe consistant en vaccin entier vivant, vaccin entier inactivé, vaccin de sous-unité, vaccin recombinant ce premier vaccin présentant l'antigène codé par le vaccin polynucléotidique ou un antigène assurant une protection croisée, pour une administration de ce premier vaccin en primo-vaccination et pour un rappel avec le vaccin polynucléotidique.

12. Vaccin selon l'une quelconque des revendications 1 à 9, accompagné d'une notice indiquant que ce vaccin polynudéotide est utilisable en rappel d'un premier vaccin choisi dans le groupe consistant en vaccin entier vivant, vaccin entier inactivé, vaccin de sous-unité, vaccin recombinant, ce premier vaccin présentant l'antigène codé par le vaccin polynucléotidique ou un antigène assurant une protection croisée.

13. Utilisation d'un plasmide tel que décrit dans l'une quelconque des revendications 1 à 9, pour la fabrication d'un vaccin destiné à vacciner les bovins contre le virus Pl-3 en association avec un vaccin entier vivant ou inactivé, recombinant ou de sous-unité dirigé contre une autre pathologie bovine.

## Patentansprüche

1. Rinderimpfstoff umfassend ein Plasmid, das ein Gen des Parainfluenza-Virus Typ 3 (PI-3) enthält, wobei das Gen ausgewählt ist aus der Gruppe bestehend aus HN und F, und einen geeigneten Träger.

2. Impfstoff nach Anspruch 1, wobei das Plasmid die zwei Gene HN und F umfasst.

3. Impfstoff nach Anspruch 1, umfassend ein Plasmid, das HN enthält, und ein Plasmid, das F enthält.

4. Impfstoff nach einem der Ansprüche 1 bis 3, wobei das Plasmid einen Promotor umfasst ausgewählt aus der Gruppe bestehend aus CMV-IE-Promotor, früher SV40-Promotor, später SV40-Promotor, LTR-Promotor des Rousschen Sarkom-Virus und Promotor eines Zytoskelettgens.

5. Impfstoff nach Anspruch 4, wobei der Promotor ein CMV-IE-Promotor ist.

6. Impfstoff nach Anspruch 4, wobei der Promotor eines Zytoskelettgens der Desmin-Promotor oder der Aktin-Promotor ist.

7. Impfstoff nach einem der Ansprüche 1 bis 6, **dadurch gekennzeichnet, dass** er außerdem ein anderes Plasmid umfasst, das ein Gen eines anderen respiratorischen Pathogens bei Rindern enthält und exprimiert.

8. Impfstoff nach einem der Ansprüche 1 bis 6, **dadurch gekennzeichnet, dass** er weiterhin zwei Plasmide enthält, die ausgewählt sind aus der Gruppe bestehend aus einem Plasmid, das das Gen gB oder gD des Rinderherpesvirus enthält, einem Plasmid, das das E2-, C+E1+E2- und E1+E2-Gen des "Mucosal-Disease"-Virus enthält und ein Plasmid, das das F- oder G-Gen des "Respiratory Syncytial"-Virus bei Rindern enthält.

9. Impfstoff nach einem der Ansprüche 1 bis 8, **dadurch gekennzeichnet, dass** er von jedem Plasmid 10 ng bis 1 mg, vorzugsweise 100 ng bis 500 µg und noch bevorzugter 1 µg bis 250 µg umfasst.

10. Verwendung eines oder mehrerer der In einem der Ansprüche 1 bis 9 beschriebenen Plasmids/e zur Herstellung eines Impfstoffes gegen das Parainfluenza-Virus Typ 3 (PI-3) für die Impfung von primär geimpften Rindern mit einem Primärimpfstoff ausgewählt aus der Gruppe bestehend aus einem Lebendvollimpfstoff, einem inaktivierten Vollimpfstoff, einem Impfstoff aus einer Untereinheit, einem rekombinanten Impfstoff, wobei der Primärimpfstoff das oder die Antigen(e) präsentiert, das/die durch das oder die Plasmid(e) oder Antigen(e) codiert wird/werden, wodurch ein Kreuzschutz gewährleistet wird.

11. Impfstoffkit, der einen Polynucleotidimpfstoff gemäß einem der Ansprüche 1 bis 9, und einen Primärimpfstoff umfasst, der ausgewählt ist aus der Gruppe bestehend aus einem Lebendvollimpfstoff, einem inaktivierten Vollimpfstoff, einem Impfstoff aus einer Untereinheit, einem rekombinanten Impfstoff, wobei der Primärimpfstoff das Antigen präsentiert, das durch den Polynucleotidimpfstoff codiert wird oder ein Antigen, wodurch ein Kreuzschutz gewährleistet wird, zur Verabreichung des Primärimpfstoffs in einer Primärimpfung und für eine Wiederauffrischung mit dem Polynucleotidimpfstoff.

12. Impfstoff gemäß einem der Ansprüche 1 bis 9 mit einem Hinweis, dass dieser Polynucleotidimptstoff bei einer Wiederauffrischung des Primärimpfstoffes verwendet werden kann, der ausgewählt ist aus der Gruppe bestehend aus einem Lebendvollimpfstoff, einem inaktivierten Vollimpfstoff, einem Impfstoff aus einer Untereinheit, einem rekombinanten Impfstoff, wobei der Primärimpfstoff das Antigen präsentiert, das durch den Polynucleotidimpfstoff oder ein Antigen codiert wird, wodurch ein Kreuzschutz gewährleistet wird.

13. Verwendung eines Plasmids wie in einem der Ansprüche 1 bis 9 beschrieben zur Herstellung eines Impfstoffes zur Impfung von Rindern gegen das PI-3-Virus zusammen mit einem Lebendvollimpfstoff oder einem inaktivierten Vollimpfstoff, einem rekombinanten Impfstoff oder einem Impfstoff aus einer Untereinhalt gegen eine andere Rinderkrankheit.

## Claims

1. A bovine vaccine comprising a plasmid containing a parainfluenza virus type 3 (PI-3) gene, said gene being selected from the group consisting of HN and F, and a suitable vehicle.

2. A vaccine according to claim 1, in which the plasmid comprises the two genes HN and F.

3. A vaccine according to claim 1, which comprises a plasmid containing HN and a plasmid containing F.

4. A vaccine according to any one of claims 1 to 3, in which the plasmid comprises a promoter selected from the group consisting of the CMV-IE promoter, the SV40 early promoter, the SV40 late promoter, the LTR promoter of the Rous sarcoma virus and a promoter for a cytoskeleton gene.

5. A vaccine according to claim 4, in which the promoter is a CMV-IE promoter.

6. A vaccine according to claim 4, in which the promoter for a cytoskeleton gene is the promoter for desmin or the promoter for actin.

7. A vaccine according to any one of claims 1 to 6, **characterized in that** it further comprises a plasmid containing and expressing a gene for another bovine respiratory pathogen.

8. A vaccine according to any one of claims 1 to 6, **characterized in that** it further comprises two plasmids selected from the group consisting of a plasmid containing the gB or gD gene of the bovine herpes virus, a plasmid containing the E2, C+E1+E2 and E1+E2 gene of the mucosal disease virus and a plasmid containing the F or G gene of bovine syncytial respiratory virus.

9. A vaccine according to any one of claims 1 to 8, **characterized in that** it comprises 10 ng to 1 mg, preferably 100 ng to 500 µg, more preferably 1 µg to 250 µg of each plasmid.

10. Use of one or more plasmids as described in any one of claims 1 to 9, for the manufacture of a vaccine intended tor the vaccination of primovaccinated cattle using a first vaccine selected from the group consisting of live whole vaccine, inactivated whole vaccine, sub-unit vaccine and recombinant vaccine, said first vaccine having the antigen or antigens encoded by the plasmid(s) or antigen(s) ensuring cross protection.

11. A vaccination kit containing a polynucleotide vaccine according to any one of claims 1 to 9 and a first vaccine selected from the group consisting of live whole vaccine, inactivated whole vaccine, sub-unit vaccine and recombinant vaccine, said first vaccine having the antigen encoded by the polynucleotide vaccine or an antigen ensuring cross protection, for administration of said first vaccine for primovaccination and as a booster with the polynucleotide vaccine.

12. A vaccine according to any one of claims 1 to 9, accompanied by a notice indicating that said polynucleotide vaccine can be used as a booster for a first vaccine selected from the group consisting of live whole vaccine, inactivated whole vaccine, sub-unit vaccine and recombinant vaccine, said first vaccine having the antigen encoded by the polynucleotide vaccine or an antigen ensuring cross protection.

13. Use of a plasmid as described in any one of claims 1 to 9, for the manufacture of a vaccine for the vaccination of cattle against PI-3 virus in association with a live or inactivated whole, recombinant or sub-unit vaccine directed against another bovine disease.
